(19) Europäisches Patentamt
European Patent Office
Office européen des brevets

(11) Publication number: **0 141 502**
**B1**

(12) **EUROPEAN PATENT SPECIFICATION**

(45) Date of publication of patent specification: **25.01.89**

(21) Application number: **84306002.1**

(22) Date of filing: **31.08.84**

(51) Int. Cl.⁴: **C 07 D 491/22**, C 08 K 5/34 // (C07D491/22, 319:00, 319:00, 221:00, 221:00)

(54) **Piperidine derivatives, their production, and polymers photostabilized thereby.**

(30) Priority: **05.09.83 JP 163734/83**
**10.05.84 JP 94371/84**
**21.05.84 JP 103362/84**

(43) Date of publication of application:
**15.05.85 Bulletin 85/20**

(45) Publication of the grant of the patent:
**25.01.89 Bulletin 89/04**

(84) Designated Contracting States:
**CH DE FR GB IT LI NL**

(56) References cited:
**US-A-4 237 294**
**US-A-4 250 268**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Takahashi, Yokoh**
**11-8-208, Sone Higashimachi 2-chome**
**Toyonaka Osaka-fu (JP)**
Inventor: **Fujii, Takeo**
**10-3-325, Sone Higashimachi 2-chome**
**Toyonaka Osaka-fu (JP)**
Inventor: **Shionoya, Masahisa**
**106-53, Wakecho**
**Izumi Osaka-fu (JP)**
Inventor: **Yachigo, Shinichi**
**11-7-305, Sone Higashimachi 2-chome**
**Toyonaka Osaka-fu (JP)**
Inventor: **Ishii, Takaki**
**1-12-2, Uchihonmachi**
**Suita Osaka-fu (JP)**

(74) Representative: **Geering, Keith Edwin**
**REDDIE & GROSE 16 Theobalds Road**
**London WC1X 8PL (GB)**

**EP 0 141 502 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to piperidine derivatives, their production and stabilized high polymer compositions containing them.

It is well known that high polymers such as polyethylene, polypropylene, polyvinyl chloride, polyurethane, and ABS resin deteriorate under the action of light, which can variously cause phenomena such as softening, embrittlement, surface cracking, and discoloration.

For preventing such deterioration, the use of various photostabilizers is well known. Such photostabilizers include for example benzophenone compounds [e.g. 2 - hydroxy - 4 - n - octoxybenzophenone], benzotriazole compounds [e.g. 2 - (2 - hydroxy - 3 - tert - butyl - 5 - methylphenyl) - 5 - chlorobenzotriazole, 2 - (2 - hydroxy - 3,5 - dipentylphenyl)benzotriazole], cyanoacrylate compounds [e.g. ethyl 2 - cyano - 3,3 - diphenylacrylate], Ni compounds [e.g. Ni salt of bis(3,5 - di - tert - butyl - 4 - hydroxybenzylphosphoric acid monoethyl ester)], and hindered piperidine compounds [e.g. 4 - benzoyloxy - 2,2,6,6 - tetramethylpiperidine, bis(2,2,6,6 - tetramethyl - 4 - piperidyl) sebacate, a reaction product of N,N' - bis(2,2,6,6 - tetramethyl - 4 - piperidylhexylenediamine) with 2,4 - dichloro - 6 - tert - octylamino - 1,3,5 - triazine]. Some of these photostabilizers are disclosed, for example, in USP 3640928, USP 3899464, USP 4086204, USP 4237294 and USP 4250268.

The photostabilizers, however, are not quite satisfactory in terms of light fastness.

It is also well known, for preventing deterioration by heat and oxidation, to use phenol compounds and sulfur compounds together with these photostabilizers. However, when the known hindered piperidine compounds, popular photostabilizers, are so used, their excellent inherent photostabilizing effect is markedly reduced, probably due to their antagonistic action on the sulfur stabilizer.

After extensive work the present inventors found that piperidine derivatives represented by the formula (I) below can reduce photo-deterioration of high polymers, and that when used together with sulfur compounds, they do not show the extreme reduction in light fastness noted with the various popular known hindered piperidine compounds.

The present invention provides photostabilizers which are piperidine derivatives or mixtures thereof selected from di- and tri-substituted sorbitol compounds of formula (I):

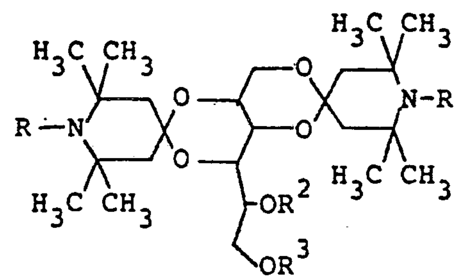

(I)

wherein $R^2$ and $R^3$ represent hydrogen atoms or taken together form

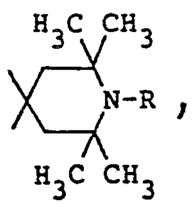

and each R is selected from a hydrogen atom and $C_1$—$C_3$ alkyl groups.

The $C_1$—$C_3$ alkyl groups include a methyl, ethyl n-propyl and isopropyl gorups.

The piperidine derivatives of the present invention represented by the foregoing formula (I), and mixtures thereof, can be produced by reacting at least one triacetonamine derivate represented by the formula (III), or a salt thereof,

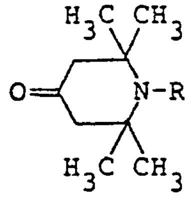

(II)

wherein R is as defined above, with sorbitol represented by the formula (III)

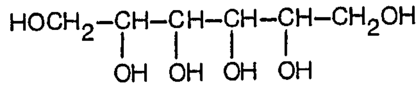 (III),

in the presence of acidic catalyst and/or dehydrating agent.

Suitable salts of the triacetonamine derivative include e.g. salts with a mineral acid (e.g. hydrochloric acid, phosphoric acid, sulfuric acid), carboxylic acid (e.g. acetic acid, oxalic acid), and sulfonic acid (e.g. p-toluenesulfonic acid).

This reaction can be carried out, with or without a solvent.

The reaction temperature is e.g. 10° to 300°C, preferably 60° to 200°C.

Suitable solvents include for example aliphatic hydrocarbons (e.g. hexane, heptane), aromatic hydrocarbons (e.g., benzene, toluene, xylene), alicyclic hydrocarbons (e.g. cyclohexane), water-soluble polar solvents (e.g. N,N-dimethylformamide, dimethyl sulfoxide, dioxane, sulfolane), alcohols (e.g. methanol, ethanol, propanol, isopropyl alcohol, butanol, tert-butanol, n-amyl alcohol, isoamyl alcohol, 2-ethylhexyl alcohol, cyclohexanol), and glycol ethers (e.g. ethylene glycol monomethyl ether, ethylene glycol monoethyl ether, ethylene glycol monobutyl ether). These solvents may be used alone or in combinations of two or more thereof.

Suitable acidic catalysts include for example hydrochloric acid, sulfuric acid, phosphoric acid, polyphosphoric acid, hydrobromic acid, p-toluenesulfonic acid, zinc chloride, boron trifluoride, cation-exchange resins, aluminum chloride/polymer complexes, selenium oxide, and ammonium chloride. Suitable dehydrating agents include calcium chloride, etc.

In this reaction of the triacetonamine derivative or its salt with sorbitol, when both $R^2$ and $R^3$ in the formula (I) are a hydrogen atom, the amount of said derivative or its salt used is generally 1.8 to 2.5 moles, preferably 1.8 to 2.2 moles based on 1 mole of sorbitol, and that of the acidic catalyst or dehydrating agent used is 0.01 to 4 moles, preferably 0.1 to 3 moles based on 1 mole of sorbitol. Also, when $R^2$ and $R^3$ in the formula (I) taken together form

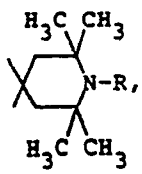

the amount of said derivative or its salt used is generally 2 to 6 moles, preferably 2.5 to 4 moles based on 1 mole of sorbitol, and that of the acidic catalyst or dehydrating agent used is 0.01 to 6 moles, preferably 0.1 to 4 moles based on 1 mole of sorbitol.

After completion of the reaction, the product can be isolated from the reaction mixture, for example by making the reaction mixture alkaline, removing the solvent from the organic layer, and if necessary recrystallizing the product from a suitable solvent.

As piperidine derivatives of the present invention thus obtainable, there are for example 1,3:2,4:5,6 - tris - O - (2,2,6,6 - tetramethyl - 4 - piperidinylidene)sorbitol, 1,3:2,4:5,6 - tris - O - (1,2,2,6,6 - pentamethyl - 4 - piperidinylidene)sorbitol, 1,3:2,4:5,6 - tris - O - (2,2,6,6 - tetramethyl - 1 - propyl - 4 - piperidinylidene)sorbitol, 1,3:2,4 - bis - O - (2,2,6,6 - tetramethyl - 4 - piperidinylidene)sorbitol, 1,3:2,4 - bis - O - (1,2,2,6,6 - pentamethyl - 4 - piperidinylidene)sorbitol, and 1,3:2,4 - bis - O - (2,2,6,6 - tetramethyl - 1 - propyl - 4 - piperidinylidene)sorbitol.

The method of the present invention can, depending upon the reaction conditions, produce a mixture of disubstituted product of sorbitol, i.e. a compound of the formula (I) in which both $R^2$ and $R^3$ are a hydrogen atom, and trisubstituted product thereof, i.e. a compound of the formula (I) in which $R^2$ and $R^3$ taken together form a hindered piperidine group. These and other mixtures of compounds according to the invention can be used as stabilizers without special problems.

When the piperidine derivatives of the present invention are used as stabilizer for high polymers, the amount blended with the high polymer is generally 0.01 to 5 parts by weight, preferably 0.05 to 2 parts by weight, based on 100 parts by weight of said high polymer. For the blending, well-known apparatus and methods for incorporating stabilizers, pigments, fillers, etc. in synthetic high polymers may be used.

In applying the piperidine derivatives of the present invention as stabilizer for high polymers, other additives such as antioxidants, photostabilizers, metal deactivating agents, metal soaps, nucleating agents, lubricants, antistatic agents, flame retardants, pigments, fillers and the like may be used together therewith.

Particularly, the thermal stability and oxidation stability of high polymers can be improved by using formula (I) compound together with phenol (preferably hindered phenol) antioxidant. Suitable phenol antioxidants include for example 2,6 - di - tert - butyl - 4 - methylphenol, n - octadecyl 3 - (3,5 - di - tert - butyl - 4 - hydroxyphenyl)propionate, 2,2' - methylenebis(4 - methyl - 6 - tert - butylphenol), 4,4' - butylidenebis(3 - methyl - 6 - tert - butylphenol), 4,4' - thiobis(3 - methyl - 6 - tert - butylphenol), 2 - tert - butyl - 6 - (3 - tert - butyl - 5 - methyl - 2 - hydroxybenzyl) - 4 - methylphenyl acrylate, 1,1,3 -

tris(2 - methyl - 4 - hydroxy - 5 - tert - butylphenyl)butane, 1,3,5 - trimethyl - 2,4,6 - tris(3 - alkyl - 5 - tert - butyl - 4 - hydroxybenzyl)benzene, 1,3,5 - tris(3 - alkyl - 5 - tert - butyl - 4 - hydroxybenzyl)-isocyanurate, 1,3,5 - tris[3 - (3 - alkyl - 5 - tert - butyl - 4 - hydroxyphenyl)propionyloxyethyl]-isocyanurate, ethylene glycol bis[3,3 - bis(4 - hydroxy - 3 - tert - butylphenyl)butanate], and pentaerythritol tetrakis[3 - (3 - alkyl - 5 - tert - butyl - 4 - hydroxyphenyl)propionate].

Sulfur antioxidants such as dilauryl thiodipropionate, dimyristyl thiodipropionate, distearyl thiodipropionate, pentaerythritol tetrakis(3 - dodecylthiopropionate), 3,9 - bis(2 - dodeceylthioethyl) - 2,4,8,10 - tetraoxaspiro[5·5]undecane may also be used together with formula (I) compound.

When piperidine derivative of the present invention is used together with phenol antioxidant and sulfur antioxidant, the weight ratio of piperidine derivative to phenol antioxidant to sulfur antioxidant is e.g. 1—20 to 1 to 1—15, and the total weight of these stabilizers is generally 0.01 to 5 parts by weight, preferably 0.05 to 2 parts by weight, based on 100 parts by weight of high polymer.

The color of high polymers can be improved by using phosphite antioxidant together with formula (I) compound.

Suitable phosphite antioxidant include for example tris(nonylphenyl)phosphite, distearyl pentaerythritol diphosphite, tris(2,4 - di - tert - butylphenyl)phosphite, tris(2 - tert - butyl - 4 - methylphenyl)phosphite, bis(2,4 - di - tert - butylphenyl)pentaerythritol diphosphite, and tetrakis(2,4 - di - tert - butylphenyl) - 4,4′ - biphenylene diphosphonite.

The light fastness of high polymers can be improved by using other photostabilizers with the hindered piperidine compounds of formula I.

Such other photostabilizers include for example benzophenone compounds such as 2 - hydroxy - 4 - methoxybenzophenone, 2 - hydroxy - n - octoxybenzophenone, 2,2′ - dihydroxy - 4,4′ - dimethoxybenzophenone; benzotriazole compounds such as 2 - (2 - hydroxy - 3 - tert - butyl - 5 - methylphenyl) - 5 - chlorobenzotriazole, 2 - (2 - hydroxy - 3,5 - dipentylphenyl)benzotriazole, 2 - (2 - hydroxy - 3 - tert - butyl - 5 - methylphenyl) - benzotriazole, 2 - (2 - hydroxy - 5 - methylphenyl)-benzotriazole, 2 - (2 - hydroxy - 3,5 - di - tert - butylphenyl)benzotriazole, 2 - (2 - hydroxy - 3,5 - di - tert - butylphenyl) - 5 - chlorobenzotriazole, 2 - (2 - hydroxy - 5 - tert - octylphenyl)benzotriazole, 2 - [2 - hydroxy - 3,5 - di - (α,α - dimethylbenzyl)phenyl]benzotriazole; benzoate compounds such as phenyl salicylate, p - tert - butylphenyl salicylate, 2,4 - di - tert - butylphenyl 3′,5′ - di - tert - butyl - 4′ - hydroxybenzoate, hexadecyl 3,5 - di - tert - butyl - 4 - hydroxybenzoate; nickel compounds such as Ni salt of dibutyldithiocarbamic acid, [2,2′ - thiobis(4 - tert - octylphenolate)] - n - butylamine nickel complex, Ni salt of bis(3,5 - di - tert - butyl - 4 - hydroxybenzylphosphoric acid monoethyl ester); cyanoacrylate compounds such as ethyl 2 - cyano - 3,3 - diphenylacrylate; and oxalic acid diamides such as N - 2 - ethylphenyl - N′ - 2 - ethoxy - 5 - tert - butylphenyloxalic acid diamide, N - 2 - ethylphenyl - N′ - 2 - ethoxyphenyloxalic acid diamide.

High polymers stabilizable by the piperidine derivatives of the present invention, include for example poly α-olefins such as low-density polyethylene, medium- and high-density polyethylenes, linear low-density polyethylene, polypropylene, polybutene-1; poly α-olefin copolymers such as propylene/ethylene random or block copolymers, ethylene/butene-1 random copolymers; poly α-olefin/vinyl monomer copolymers such as maleic acid anhydride-modified polypropylene; chlorinated polyethylene; EVA resin; polyvinyl chloride; methacrylic resin; polystyrene; high impact polystyrene; ABS resin; AES resin; MBS resin; polyethylene terephthalate; polybutylene terephthalate; polyamide; polyimide; polycarbonate; polyacetal; polyurethane; unsaturated polyester resin; rubbers such as isoprene rubber, butadiene rubber; acrylonitrile/butadiene copolymer rubber, styrene/butadiene copolymer rubber; and blends of these polymers.

The invention is illustrated in detail by the following Examples, which in no way limit the invention.

## Example 1

To a four-necked flask equipped with a thermometer, a stirrer and Dean-Stark trap were added 100 g (0.52 mole) of triacetonamine hydrochloride, 20.94 g (0.12 mole) of sorbitol, 300 g of toluene and 100.8 g of p-toluenesulfonic acid monohydrate, and the mixture was heated with stirring and reacted at 110°C to 120°C for 3 hours.

During this period, formed water was removed from the reaction system by means of the Dean-Stark trap.

After completion of the reaction, an aqueous sodium hydroxide solution was added to the reaction solution to make the product soluble in toluene. The toluene layer containing the dissolved product was separated, washed with water and dried, and toluene was removed by evaporation to obtain 51.89 g of a yellowish brown and glassy 1,3:2,4:5,6-tris-O-(2,2,6,6-tetramethyl-4-piperidinylidene)sorbitol (yield, 73% based on sorbitol).

This yellowish brown and glassy product was recrystallized from a hexane/water mixture to obtain 25.92 g of white crystals (m.p., 43—46°C).

FD-mass spectrometry:

A parent ion peak 593 was confirmed.

$^1$H—NMR ($CDCl_3$, $D_2O$): δ (ppm):

1.22 (36H, s), 1.55 (12H, m), 4.05 (8H, m)

$^{13}C$—NMR δ (ppm):

31.106 (off resonance, q), 31.261 (q), 31.839 (q), 32.058 (q), 32.220 (q), 32.315 (q), 32.379 (q), 32.681 (q), 32.900 (q), 44.753 (t), 45.342 (45.968 (t), 46.388 (t), 46.614 (t), 46.921 (t), 51.342 (s), 65.309 (t), 67.386 (t), 74.666 (d), 76.656 (d), 77.717 (d), 79.238 (d), 110.031 (s), 110.273 (s), 110.477 (s).

Example 2

To the same flask as used in Example 1 were added 94.1 g (0.6 mole) of triacetonamine, 36.4 g (0.2 mole) of sorbitol, 500 g of xylene and 149.5 g of p-toluenesulfonic acid monohydrate, and the mixture was reacted at 135° to 140°C for 4 hours.

After completion of the reaction, after-treatment and purification were carried out in the same manner as in Example 1 to obtain 73.2 g of a yellowish brown and glassy 1,3:2,4:5,6-tris-O-(2,2,6,6-tetramethyl-4-piperidinylidene)sorbitol (yield, 62% based on sorbitol).

This product was recrystallized from a hexane/water mixture to obtain 61.7 g of white crystals (yield, 52% based on sorbitol).

From the results of melting point, elementary analysis and $^{1}H$—NMR, it was confirmed that this crystal was the same compound as obtained in Example 1.

Example 3

To the same flask as used in Example 1 were added 102.4 g (0.6 mole) of 1,2,2,6,6-pentamethyl-4-piperidone, 36.4 g (0.2 mole) of sorbitol, 500 g of xylene and 123.6 g of p-toluenesulfonic acid monohydrate, and the mixture was reacted and after-treated in the same manner as in Example 2 to obtain 87.8 g of 1,3:2,4:5,6-tris-O-(1,2,2,6,6-pentamethyl-4-piperidinylidene)sorbitol as a pale yellow and viscous liquid (yield, 69% based on sorbitol).

FD-mass spectrometry:

A parent ion peak 635 was confirmed.

$^{1}H$—NMR ($CDCl_3$, 60MHz): δ (ppm):

1.15 (36H, s), 1.54 (12H, m), 2.24 (9H, s), 4.04 (8H, m).

Example 4

To a four-necked flask equipped with a thermometer, a stirrer and a condenser were added 100 g (0.52 mole) of triacetonamine hydrochloride, 45.5 g (0.25 mole) of sorbitol and 300 g of toluene, and thereafter, 100.8 g of p-toluenesulfonic acid monohydrate was added with stirring. The mixture was heated to 110°C and reacted at this temperature for 1 hour.

After completion of the reaction, an aqueous sodium hydroxide solution was added to the reaction solution to make the product soluble in toluene. The toluene layer containing the dissolved product was separated, washed with water and dried, and toluene was removed by evaporation to obtain 71 g of 1,3:2,4-bis-O-(2,2,6,6-tetramethyl-4-piperidinylidene)sorbitol as yellow crystals (yield, 62.3% based on sorbitol).

The yellow crystals were dissolved in toluene and treated with activated carbon to obtain pale yellow crystals. M.p., 167—173°C.

A parent ion peak 456 was confirmed by FD—MS.

$^{1}H$—NMR DMSO $D_2O$, TSP): δ (ppm):

1.16 (24H, s), 1.53 (8H, s), 3.66 (5H, d), 4.00 (3H, s).

Example 5

To the same flask as used in Example 1 were added 80.7 g (0.52 mole) of triacetonamine, 45.5 g (0.25 mole) of sorbitol and 300 g of toluene, and thereafter, 100.8 g of p-toluenesulfonic acid monohydrate was added with stirring. The mixture was heated to 110°C and reacted at this temperature for 1-hour.

After completion of the reaction, the reaction solution was after-treated in the same manner as in Example 1 to obtain 68 g of 1,3:2,4-bis-O-(2,2,6,6-tetramethyl-4-piperidinylidene)sorbitol as yellow crystals (yield, 60% based on sorbitol). The physical properties of this product were the same as those of the product obtained in Example 4.

Example 6

To the same flask as used in Example 1 were added 102.4 g (0.6 mole) of 1,2,2,6,6-pentamethyl-4-piperidone, 54.7 g (0.3 mole) of sorbitol and 300 g of xylene, and thereafter, 116.0 g of p-toluenesulfonic acid monohydrate was added with stirring. The mixture was heated to 120°C and reacted at this temperature for 1 hour.

After completion of the reaction, after-treatment and purification were carried out in the same manner as in Example 1 to obtain 110.4 g of a pale yellow and oily 1,3:2,4-bis-O-(1,2,2,6,6-pentamethyl-4-piperidinylidene)sorbitol (yield, 76% based on sorbitol).

$M^{+}1$ (485) was confirmed by FD—MS.

$^{1}H$—NMR (DMSO, $D_2O$, TSP): δ (ppm):

1.11 (24H, s), 1.52 (8H, s), 2.22 (6H, s), 3.63 (5H, d), 4.03 (3H, s).
Elementary analysis (for $C_{26}H_{48}N_2O_6$):

| | Found | Calculated |
|---|---|---|
| C | 64.00% | 64.43% |
| H | 9.72% | 9.98% |
| N | 5.00% | 5.78% |

Example 7

The components listed below were mixed on a mixer for 5 minutes and then melt-kneaded at 180°C on a mixing roll to obtain a blend. This blend was then formed on a hot press kept at 120°C into a sheet 1 mm thick from which a test piece 150 × 30 × 1 mm in size was prepared. This was repeated using different test compounds.

Each test piece was exposed to light in a Sunshine weather-O-meter (light source, carbon arc; temperature of black panel, 83 ± 3°C; spraying cycle, 120 minutes; spraying time, 18 minutes) and folded every 60 hours to obtain the time required for the test piece to break in two. The weathering resistance was evaluated by this time.

The results are shown in Table 1.

Blend:

| | Part by weight |
|---|---|
| Unstabilized polypropylene | 100 |
| Calcium stearate | 0.1 |
| 2,6-Di-tert-butyl-4-methylphenol | 0.05 |
| Test compound | 0.2 |

In Tables 1 to 5 UVA—1 to UVA—9 and I—1 to I—4 mean the following compounds:

UVA—1: 2-Hydroxy-4-methoxybenzophenone
UVA—2: 2-Hydroxy-4-n-octoxybenzophenone
UVA—3: 2-(2-Hydroxy-5-methylphenyl)benzotriazole
UVA—4: 2-(2-Hydroxy-3-tert-butyl-5-methylphenyl)-5-chlorobenzotriazole
UVA—5: 2-(2-Hydroxy-3,5-dipentylphenyl)benzotriazole
UVA—6: Ethyl 2-cyano-3,3′-diphenylacrylate
UVA—7: Nickel salt of bis(3,5-di-tert-butyl-4-hydroxybenzylphosphoric acid) monoethyl ester
UVA—8: Bis(2,2,6,6-tetramethyl-4-piperidyl)sebacate
UVA—9: Bis-O-(2,2,6,6-tetramethyl-4-piperidinylidene)pentaerythritol
I—1: 1,3:2,4:5,6-Tris-O-(2,2,6,6-tetramethyl-4-piperidinylidene)sorbitol
I—2: 1,3:2,4:5,6-Tris-O-(1,2,2,6,6-pentamethyl-4-piperidinylidene)sorbitol
I—3: 1,3:2,4-Bis-O-(2,2,6,6-tetramethyl-4-piperidinylidene)sorbitol
I—4: 1,3:2,4-Bis-O-(1,2,2,6,6-pentamethyl-4-piperidinylidene)sorbitol

Table 1

| | No. | Test compound | Weathering resistance (hours) |
|---|---|---|---|
| Example | 1 | I - 1 | 2120 |
| | 2 | I - 2 | 2050 |
| | 3 | I - 3 | 2100 |
| | 4 | I - 4 | 2040 |
| Comparative example | 5 | UVA - 1 | 180 |
| | 6 | UVA - 2 | 420 |
| | 7 | UVA - 3 | 240 |
| | 8 | UVA - 4 | 600 |
| | 9 | UVA - 5 | 420 |
| | 10 | UVA - 6 | 240 |
| | 11 | UVA - 7 | 360 |
| | 12 | UVA - 8 | 1800 |
| | 13 | UVA - 9 | 1680 |
| | 14 | No addition | 120 |

Example 8

To respective lots of 25% urethane dope (comprising 25 parts of a polyurethane resin, 3.75 parts of dimethylformamide and 71.25 parts of tetrahydrofuran) were added to the test compounds shown in Table 2 at a rate of 1 wt.% based on the polyurethane resin. Each mixture was coated onto polyester film in a thickness of 1.2 mm and dried for 1 hour in a dryer kept at 45°C. The sheets thus obtained were punched into No. 3 dumb-bell test pieces. The test pieces were exposed to light for 60 hours and 120 hours in a fade-O-meter (light source, ultraviolet carbon arc; temperature of black panel, 63 ± 3°C), and the retention of break strength was obtained by the tensile test (tensile rate, 200 mm/mm; measurement temperature, 25°C).

The results are shown in Table 2.

Table 2

| | No. | Test compound | Percent retention of break strength | |
|---|---|---|---|---|
| | | | 60 hrs. | 120 hrs. |
| Example | 1 | I - 1 | 80 | 58 |
| | 2 | I - 2 | 78 | 56 |
| | 3 | I - 3 | 79 | 58 |
| | 4 | I - 4 | 77 | 55 |
| Comparative example | 5 | UVA - 2 | 43 | 22 |
| | 6 | UVA - 5 | 56 | 30 |
| | 7 | UVA - 8 | 66 | 40 |
| | 8 | No addition | 30 | 16 |

Example 9

The blend described below was melt-kneaded on a mixing roll kept at 150°C and then formed into a sheet 0.5 mm thick on a hot press kept at 160°C. This was repeated using different test compounds.

Each sheet was exposed to light for 1200 hours in Sunshine weather-O-meter (light source, carbon arc; temperature of black panel, 63 ± 3°C; spraying cycle, 120 minutes; spraying time, 18 minutes), and the degree of discoloration was observed.

The results are shown in Table 3.

Blend:

| | Parts by weight |
|---|---|
| Polyvinyl chloride | 100 |
| Dioctyl phthalate | 38 |
| Epoxidized soybean oil | 2 |
| Barium stearate | 1 |
| Zinc stearate | 0.3 |
| Test compound | 0.2 |

Table 3

| | No. | Test compound | Color |
|---|---|---|---|
| Example | 1 | I - 1 | Pale yellow |
| | 2 | I - 2 | Pale yellow |
| | 3 | I - 3 | Pale yellow |
| | 4 | I - 4 | Pale yellow |
| Comparative example | 5 | UVA - 2 | Brown spots |
| | 6 | UVA - 3 | Yellow |
| | 7 | UVA - 8 | Yellow |
| | 8 | No addition | Blackish brown |

Example 10

The components below were mixed on a mixer for 5 minutes and then melt-kneaded at 180°C on a mixing roll to obtain a blend. This blend was then formed on a hot press kept at 210°C into a sheet 1 mm thick and a test piece 150 × 30 × 1 mm in size was prepared. This was repeated using different test compounds.

Each test piece was exposed to light in a Sunshine weather-O-meter (light source, carbon arc; temperature of black panel, 83 ± 3°C; spraying cycle, 120 minutes; spraying time, 18 minutes) and folded every 60 hours to obtain the time required for the test piece to break in two. The weathering resistance was evaluated by this time.

Separately from this, test pieces 40 × 40 × 1 mm in size were prepared and placed in a Geer oven kept at 160°; in each case the time required for 30% of the test piece area to become brittle was measured and taken as the thermal brittleness induction period to evaluate thermal and oxidation stability.

The results are shown in Table 4.

Blend:

| | Part by weight |
|---|---|
| Unstabilized polypropylene | 100 |
| Calcium stearate | 0.01 |
| 2,6-Di-tert-butyl-4-methylphenol | 0.05 |

| | | |
|---|---|---|
| Test compound | photostabilizer | 0.2 |
| | phenol compound | 0.05 |
| | sulfur compound | 0.25 |

In Table 4 UVA—10 and AO—1 to AO—3 mean the following compounds:

UVA—10: Tinuvin 944 (produced by Ciba-Geigy Co.) (hindered piperidine photostabilizer)

AO—1: Tris(3,5-di-tert-butyl-4-hydroxybenzyl)isocyanurate

AO—2: Dilauryl 3,3′-thiodipropionate

AO—3: Pentaerythritol tetrakis(3-dodecylthiopropionate)

**Table 4**

| | No. | Photo-stabilizer | Phenol compound | Sulfur compound | Light fastness (hours) | Thermal brittleness induction period (hours) |
|---|---|---|---|---|---|---|
| Example | 1 | I-1 | AO-1 | AO-2 | 1860 | 710 |
| | 2 | I-2 | do | do | 1800 | 685 |
| | 3 | I-3 | do | do | 1800 | 680 |
| | 4 | I-4 | do | do | 1740 | 670 |
| | 5 | I-1 | do | AO-3 | 1860 | 690 |
| | 6 | I-2 | do | do | 1800 | 660 |
| | 7 | I-3 | do | do | 1800 | 660 |
| | 8 | I-4 | do | do | 1740 | 650 |
| Comparative example | 9 | UVA-2 | do | AO-2 | 480 | 490 |
| | 10 | UVA-4 | do | do | 720 | 500 |
| | 11 | UVA-5 | do | do | 480 | 490 |
| | 12 | UVA-6 | do | do | 240 | 480 |
| | 13 | UVA-7 | do | do | 240 | 485 |
| | 14 | UVA-8 | do | do | 960 | 485 |
| | 15 | UVA-10 | do | do | 900 | 480 |
| | 16 | UVA-9 | do | do | 900 | 485 |
| | 17 | UVA-2 | do | AO-3 | 480 | 480 |
| | 18 | UVA-4 | do | do | 680 | 480 |
| | 19 | UVA-5 | AO-1 | AO-3 | 420 | 480 |
| | 20 | UVA-6 | do | do | 240 | 470 |
| | 21 | UVA-7 | do | do | 420 | 475 |
| | 22 | UVA-8 | do | do | 900 | 475 |
| | 23 | UVA-10 | do | do | 840 | 470 |
| | 24 | UVA-9 | do | do | 840 | 475 |

| | No. | Photo-stabilizer | Phenol compound | Sulfur compound | Light fastness (hours) | Thermal brittleness induction period (hours) |
|---|---|---|---|---|---|---|
| Comparative example | 25 | UVA-4 | - | - | 600 | 45 |
| | 26 | UVA-5 | - | - | 420 | 30 |
| | 27 | UVA-6 | - | - | 240 | 20 |
| | 28 | UVA-7 | - | - | 360 | 30 |
| | 29 | UVA-8 | - | - | 1800 | 30 |
| | 30 | UVA-10 | - | - | 1680 | 30 |
| | 31 | UVA-9 | - | - | 1680 | 30 |
| | 32 | I-1 | - | - | 2160 | 60 |
| | 33 | I-2 | - | - | 2100 | 55 |
| | 34 | I-3 | - | - | 2100 | 55 |
| | 35 | I-4 | - | - | 2040 | 55 |
| | 36 | - | AO-1 | AO-2 | 120 | 450 |
| | 37 | - | do | AO-3 | 120 | 430 |
| | 38 | No addition | | | 120 | 5 |

Example 11

To respective lots of 25% urethane dope (comprising 25 parts of polyurethane resin, 3.75 parts of dimethylformamide and 71.25 parts of tetrahydrofuran) were added the test compounds of the composition described below at a rate of 1% based on the polyurethane resin. Each mixture was coated onto polyester film in a thickness of 1.2 mm and dried for 1 hour in a dryer kept at 45°C. The sheets thus obtained were punched into No. 3 dumb-bell test pieces. The test pieces were exposed to light for 60 hours and 120 hours in a fade-O-meter (light source, ultraviolet carbon arc; temperature of black panel, 63 ± 3°C), and the percent retention of break strength was obtained by the tensile test (tensile rate, 200 mm/mm; measurement temperature, 25°C).

The results are shown in Table 5.

Test compound:
- photostabilizer 0.7 part
- phenol compound 0.05 part
- sulfur compound - 0.25 part

Table 5

| | No. | Photo-stabilizer | Phenol compound | Sulfur compound | Percent retention of break strength | |
|---|---|---|---|---|---|---|
| | | | | | 60 hrs | 120 hrs |
| Example | 1 | I-1 | AO-1 | AO-2 | 76 | 55 |
| | 2 | I-2 | do | do | 74 | 53 |
| | 3 | I-3 | do | do | 75 | 55 |
| | 4 | I-4 | do | do | 73 | 52 |
| Comparative example | 5 | UVA-2 | do | do | 41 | 21 |
| | 6 | UVA-5 | do | do | 53 | 28 |
| | 7 | UVA-8 | do | do | 56 | 33 |
| | 8 | – | do | do | 35 | 18 |
| | 9 | No addition | | | 30 | 16 |

## Claims

1. Piperidine derivatives or mixtures thereof selected from di- and tri-substituted sorbitol compounds of formula (I)

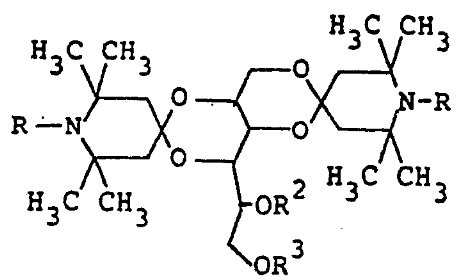

(I)

wherein $R^2$ and $R^3$ represent hydrogen atoms or taken together form

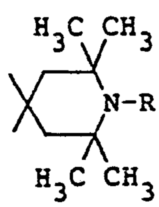

,

and each R is selected from a hydrogen atom and $C_1$—$C_3$ alkyl groups.

2. Piperidine derivatives or mixtures thereof according to claim 1 wherein R is a methyl group.

3. A method for producing piperidine derivatives or mixtures thereof selected from di- and tri-substituted sorbitol compounds as claimed in claim 1, which comprises reacting sorbitol with at least one triacetonamine derivative represented by the formula (II) or a salt thereof

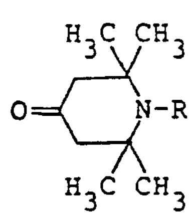

(II)

wherein R is as defined in claim 1, in the presence of acidic catalyst and/or dehydrating agent.

4. A method according to claim 3 which is conducted in the presence of a solvent, preferably comprising at least one member selected form aromatic hydrocarbons, aliphatic hydrocarbons, alicyclic

hydrocarbons, water-soluble polar solvents, alcohols and glycol ethers.

5. A method according to claim 4 for producing piperidine derivatives or mixtures thereof comprising a di-substituted sorbitol compound of formula (I) wherein there is used, per mole of sorbitol, 1.8 to 2.5 (preferably 1.8 to 2.2) moles of formula (II) compound and 0.01 to 4 (preferably 0.1 to 3) moles of acidic catalyst.

6. A method according to claim 4 for producing piperidine derivatives or mixtures thereof comprising a tri-substituted sorbitol compound of formula (I) wherein there is used, per mole of sorbitol, 2 to 6 (preferably 2.5 to 4) moles of formula (II) compound and 0.01 to 6 (preferably 0.1 to 4) moles of acidic catalyst.

7. A method according to any of claims 3 to 6 wherein the reaction temperature is 10° to 300°C, preferably 60° to 200°C.

8. Polymer material, selected for example from polyolefins, polyurethanes and polyvinyl chlorides, incorporating as a stabilizer at least one compound according to claim 1 or 2.

9. A polymer composition according to claim 8 containing phenol (preferably hindered phenol) antioxidant — e.g. 2,6-di-tert-butyl-4-methylphenol and/or tris(3,5-di-tert-butyl-4-hydroxybenzyl)isocyanurate — and sulfur antioxidant — e.g. selected from dilauryl thiodipropionate, dimyristyl thiodipropionate, distearyl thiodipropionate and pentaerythritol tetrakis(3-dodecylthiopropionate).

10. A polymer composition according to claim 9 wherein the weight ratio of formula (I) compound to phenol antioxidant to sulfur antioxidant is 1—20 to 1 to 1—15, and the totel weight of these components is 0.01 to 5 parts by weight per 100 parts by weight of polymer.

**Patentansprüche**

1. Piperidinderivate oder Gemische davon, ausgewählt aus den di- und tri-substituierten Sorbitverbindungen der Formel (I)

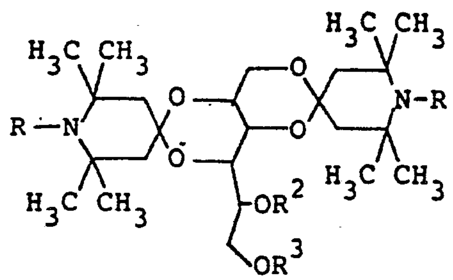

(I)

worin $R^2$ und $R^3$ Wasserstoffatome bedeuten oder zusammengenommen die Gruppe

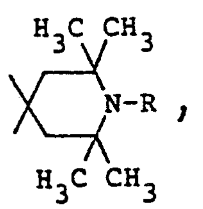

bilden und jedes R eine Wasserstoffatom oder eine $C_1$—$C_3$-Alkylgruppe darstellen kann.

2. Piperidinderivate oder Gemische davon nach Anspruch 1, dadurch gekennzeichnet, daß R eine Methylgruppe ist.

3. Verfahren zur Herstellung von Piperidinderivaten oder Gemichen davon, ausgewählt aus di- und tri-substituierten Sorbitverbindungen gemäß Anspruch 1, dadurch gekennzeichnet, daß man Sorbit mit wenigstens einem Triacetonaminderivat der Formel (II) oder einem Salz davon

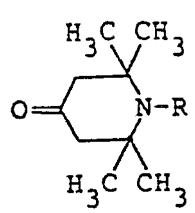

(II)

worin R die in Anspruch 1 angegebene Bedeutung hat, in Gegenwart eines sauren Katalysators und/oder eines Dehydratisierungsmittels umsetzt.

4. Verfahren nach Anspruch 3, dadurch gekennzeichnet, daß man es in Gegenwart eines Lösungsmittels, das vorzugsweise zumindest ein Mitglied der Gruppe aromatische Kohlenwasserstoffe, aliphatische Kohlenwasserstoffe, alizyklische Kohlenwasserstoffe, wasserlösliche polare Lösungsmittel,

Alkohole und Glykoläther umfaßt, durchführt.

5. Verfahren nach Anspruch 4 zur Erzeugung von Piperidinderivaten oder Gemischen davon, welche eine di-substituierte Sorbitverbindung der Formel (I) enthalten, dadurch gekennzeichnet, daß man pro Mol Sorbit 1,8 bis 2,5 (vorzugsweise 1,18 bis 2,2) Mol der Verbindung der Formel (II) und 0,01 bis 4 (vorzugsweise 0,1 bis 3) Mol von saurem Katalysator einsetzt.

6. Verfahren nach Anspruch 4 zur Herstellung von Piperidinderivaten oder Gemischen davon, welche eine tri-substituierte Sorbitverbindung der Formel (I) aufweisen, dadurch gekennzeichnet, daß man pro Mol Sorbit 2 bis 6 (vorzugsweise 2,5 bis 4) Mol der Verbindung der Formel (II) und 0,01 bis 6 (vorzugsweise 0,1 bis 4) Mol sauren Katalysator einsetzt.

7. Verfahren nach einem der Ansprüche 3 bis 6, dadurch gekennzeichnet, daß die Reaktionstemperatur 10 bis 300°C, vorzugsweise 60 bis 200°C beträgt.

8. Polymermaterial, ausgewählt z.B. aus Polyolefinen, Polyurethanen und Polyvinylchloriden, enthaltend als Stabilisator wenigstens eine Verbindung gemäß Anspruch 1 oder 2.

9. Polymerzusammensetzung gemäß Anspruch 8, enthaltend Phenol- (vorzugsweise gehindertes Phenol-) antioxidans, z.B. 2,6-Di-tert-butyl-4-methylphenol und/oder Tris(3,5-di-tert-butyl-4-hydroxybenzyl)isocyanurat und Schwefelantioxidans, z.B. ausgewählt aus Dilaurylthiodipropionat, Dimyristylthiodipropionat, Distearylthiodipropionat und Pentaerythrittetrakis(3-dodecylthiopropionat).

10. Polymerzusammensetzung nach Anspruch 9, dadurch gekennzeichnet, daß das Gewichtsverhältnis von Verbindung der formel (I) zu Phenolantioxidans zu Schwefeloxidans 1—20 zu 1 zu 1—15 beträgt und das Gesamtgewicht dieser Komponenten 0,01 zu 5 Gew.-Teilen pro 100 Gew.Teile Polymer ist.

**Revendications**

1. Dérivés de pipéridine ou leurs mélanges choisis entre des composés disubstitués et trisubstitués de sorbitol, de formule (I)

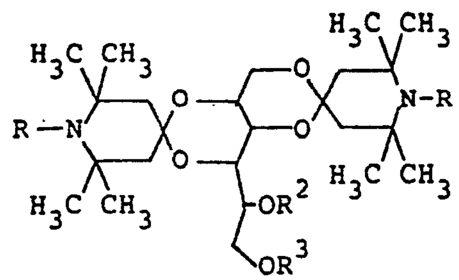

(I)

dans laquelle $R^2$ et $R^3$ représentent des atomes d'hydrogène ou forment conjointement un groupe

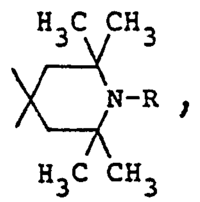

et chaque R est choisi entre un atome d'hydrogène et des groupes alkyle en $C_1$ à $C_3$.

2. Dérivés de pipéridine ou leurs mélanges suivant la revendication 1, dans lesquels R est un groupe méthyle.

3. Procédé de production de dérivés de pipéridine ou de leurs mélanges choisis entre des composés disubstitués et trisubstitués de sorbitol suivant la revendication 1, qui consiste à faire réagir du sorbitol avec au moins un dérivé de triacétonamine représenté par la formule (I) ou un sel de ce dérivé

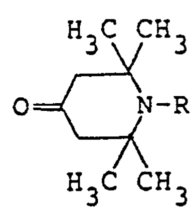

(II)

formule dans laquelle R est tel que défini dans la revendication 1, en présence d'un catalyseur acide et/ou d'un agent déshydratant.

4. Procédé suivant la revendication 3, qui est mis en oeuvre en présence d'un solvant, comprenant avantageusement au moins un représentant du groupe constitué d'hydrocarbures aromatiques, d'hydrocarbures aliphatiques, d'hydrocarbures alicycliques, de solvants polaires hydrosolubles, d'alcools et d'éthers de glycols.

5. Procédé suivant la revendication 4, pour la production de dérivés de pipéridine ou de leurs mélanges comprenant un composé de sorbitol disubstitué de formule (I), dans lequel on utilise, par mole de sorbitol, 1,8 à 2,5 (de préférence 1,8 à 2,2) moles de composé de formule (II) et 0,01 à 4 (de préférence 0,1 à 3) moles de catalyseur acide.

6. Procédé suivant la revendication 4 pour la production de dérivés de pipéridine ou de leurs mélanges comprenant un composé trisubstitué de sorbitol de formule (I), dans lequel on utilise, par mole de sorbitol, 2 à 6 (de préférence 2,5 à 4) moles de composé de formule (II) et 0,01 à 6 (de préférence 0,1 à 4) moles de catalyseur acide.

7. Procédé suivant l'une quelconque des revendications 3 à 6, dans lequel la température de réaction va de 10 à 300°C, de préférence de 60 à 200°C.

8. Matière polymérique, choisie, par exemple entre des polyoléfines, des polyuréthannes et des polychlorures de vinyle, renfermant comme agent stabilisant au moins un composé suivant la revendication 1 ou 2.

9. Composition polymérique suivant la revendication 8, contenant un agent anti-oxydant phénolique (de préférence un phénol à encombrement stérique) — par exemple du 2,6-di-tertio-butyl-4-méthylphénol et/ou de l'isocyanurate de tris-(3,5-di-tertio-butyl-4-hydroxybenzyle) — et un agent anti-oxydant contenant du soufre — par exemple choisi entre le thiodipropionate de dilauryle, le thiodipropionate de dimyristyle, le thiodipropionate de distéaryle et le tétrakis-(3-dodécylthiopropionate) de pentaérythritol.

10. Composition polymérique suivant la revendication 9, dans laquelle le composé de formule (I), l'agent anti-oxydant phénolique et l'agent anti-oxydant sulfuré sont utilisés dans des proportions de 1—20:1:1—15 et le poids total de ces composants est de 0,01 à 5 parties en poids pour 100 parties en poids de polymère.